# EUROPEAN PATENT APPLICATION

(11) **EP 2 412 366 A1**
(43) Date of publication of application: **01.02.2012**
(21) Application number: 10756118.5
(22) Date of filing: 24.03.2010
(51) Int. Cl.: A61K 9/20, A61K 9/70, A61K 47/12, A61K 47/18, A61K 47/32

(54) **SOLID PREPARATION**

(30) Priority: 25.03.2009 JP 2009074161
(71) Applicant: LINTEC Corporation, Tokyo 173-0001 (JP); ASKA Pharmaceutical Co., Ltd., Tokyo 108-8532 (JP)
(72) Inventor: TAKANO Youichi, Tokyo 173-0001 (JP); SUGIURA Yusaku, Tokyo 173-0001 (JP)
(74) Representative: von Kreisler Selting Werner
(86) International application number: PCT/JP2010/055094
(87) International publication number: WO 2010/110322

(57) **Abstract**

A solid preparation 1 comprises a drug-containing unit 2 containing a cationic drug, and a gel-forming layer 4, containing an anionic polymer, for covering the drug-containing unit 2 and forming a gel with absorbing water, and optionally an intermediate layer 3 interposed between the drug-containing unit 2 and the gel-forming layer 4. The solid preparation 1 improves the elution property of the drug by incorporating an acidic component (such as tartaric acid or citric acid) into the intermediate layer 2 and/or the intermediate layer 3. The gel-forming layer 4 may comprise a carboxyvinyl polymer and a polyvalent metal compound. The gel-forming layer 4 may be covered with a surface layer (anti-adhesive layer) 5.

## Description

### TECHNICAL FIELD

The present invention relates to a solid preparation (particularly, a solid preparation suitable for oral administration) and a method for improving an elution property (or dissolution rate) of a drug from the solid preparation.

### BACKGROUND ART

As an oral administration preparation, for example, a solid preparation in the form of a solid and a semisolid preparation in the form of a jelly (or a gel) are known. The solid preparation (for example, a tablet and a capsule) is usually difficult to swallow as it is, and is usually taken with a large quantity of water. In particular, it is often difficult for elderly people and infants to swallow the solid preparation. Moreover, the solid preparation has a risk of blocking the respiratory tract by accident or a risk of adhering to the esophagus.

On the other hand, the semisolid preparation is easy to swallow because of the jelly form thereof and is also easily administered to elderly people and infants. However, since the semisolid preparation contains a large quantity of water, the semisolid preparation confronts a problem that a drug contained in the preparation is easily decomposed or changed in quality.

In order to solve such a problem, WO 2002/087622 (Patent Document 1) and WO 2005/097080 (Patent Document 2) describes an oral administration preparation (or a film-shaped preparation) comprising a water-swellable gel-forming layer which absorbs saliva and swells to form a gel, as an outermost layer which covers a drug-containing layer. Since the water-swellable gel-forming layer absorbs water to form the gel layer, this solid preparation can be taken with a small quantity of water. However, when the water-swellable gel-forming layer is formed with an anionic or acidic polymer and the drug-containing layer contains a cationic or basic drug, the elution property (or dissolution rate) of the drug is lowered.

### RELATED ART DOCUMENTS

### PATENT DOCUMENTS

Patent Document 1: WO 2002/087622 (Claims)
Patent Document 2: WO 2005/097080 (Claims)

### SUMMARY OF THE INVENTION

### PROBLEMS TO BE SOLVED BY THE INVENTION

It is therefore an object of the present invention to provide a solid preparation having an improved elution property (or dissolution rate) of a drug even when an anionic or acidic polymer is contained in a water-swellable gel-forming layer and a cationic or basic drug is contained in a drug-containing layer, and a method for improving an elution property of the drug.

### MEANS TO SOLVE THE PROBLEMS

The inventors of the present invention made intensive studies to achieve the above objects and finally found the following facts (i) and (ii): (i) when an anionic or acidic polymer is contained in a water-swellable gel-forming layer and a cationic or basic drug is contained in a drug-containing layer, the drug is adsorbed on the gel-forming layer and the elution property of the drug from the preparation is deteriorated, and (ii) use of an acidic component in a preparation containing an anionic or acidic polymer and a cationic or basic drug prevents the adsorption of the drug effectively and improves the elution property of the drug from the preparation. The present invention was accomplished based on the above findings.

That is, the solid preparation of the present invention comprises a drug-containing unit containing a drug, a gel-forming layer for covering the drug-containing unit and forming a gel with absorbing water, and an intermediate layer interposed between the drug-containing unit and the gel-forming layer. In this solid preparation, the drug-containing unit contains a cationic or basic drug (hereinafter, the cationic or basic drug may be referred to as a cationic drug generically), the gel-forming layer contains an anionic or acidic polymer (hereinafter, the anionic or acidic polymer may be referred to as an anionic polymer generically), the intermediate layer contains a pharmaceutically acceptable acidic component. Moreover, according to another embodiment, the solid preparation comprises a drug-containing unit containing a drug, and a gel-forming layer for covering the drug-containing unit and forming a gel with absorbing water. The drug-containing unit of the solid preparation contains a cationic drug and a pharmaceutically acceptable acidic component, and the gel-forming layer of the preparation contains an anionic polymer.

The cationic drug may comprise an active component (or an active substance) having at least one basic group selected from the group consisting of a primary amino group, a secondary amino group, a tertiary amino group, and a basic nitrogen-containing heterocyclic group, or a salt thereof. Moreover, the gel-forming layer may comprise a water-soluble (meth)acrylic polymer having a carboxyl group or a salt thereof and a crosslinking agent. For example, the gel-forming layer may be formed with a carboxyvinyl polymer and a polyvalent metal compound. The acidic component may have an acidic dissociation constant pKa of not more than 4. 0. The acidic component may comprise at least one member selected from the group consisting of an inorganic acid, an aliphatic polyvalent carboxylic acid, a hydroxycarboxylic acid, and an amino acid. For example, the acidic component may comprise at least one member selected from the group consisting of succinic acid, maleic acid, fumaric acid, lactic acid, malic acid, tartaric acid, citric acid, and gluconic acid.

The ratio of the acidic component may be about 1 to 5000 parts by mass relative to 100 parts by mass of the cationic drug.

The solidpreparationmay further comprise a surface layer for covering the gel-forming layer directly or indirectly and dissolving in water to prevent adhesion of the solid preparation to an inner wall of an oral cavity (or a buccal cavity). Such a solid preparation may be a film-covered preparation (or a laminate preparation).

The present invention also includes a method for improving an elution property of a drug from the following solid preparation (1) or (2): (1) a solid preparation comprising a drug-containing unit containing a cationic drug, a gel-forming layer for covering the drug-containing unit and forming a gel with absorbing water, wherein the gel-forming layer contains an anionic polymer, and an intermediate layer interposed between the drug-containing unit and the gel-forming layer, or (2) a solid preparation comprising a drug-containing unit containing a cationic drug and a gel-forming layer for covering the drug-containing unit and forming a gel with absorbing water, wherein the gel-forming layer contains an anionic polymer; wherein the method comprises incorporating a pharmaceutically acceptable acidic component into the intermediate layer or the drug-containing unit.

### EFFECTS OF THE INVENTION

According to the present invention, even if the solid preparation comprises the water-swellable gel-forming layer containing the anionic polymer and the drug-containing unit (or layer) containing the cationic drug, the coexistence of the acidic component with the anionic polymer and the cationic drug in the solid preparation can prevent the adsorption of the drug on the anionic polymer to improve the elution property of the drug from the solid preparation.

### BRIEF DESCRIPTION OF DRAWINGS

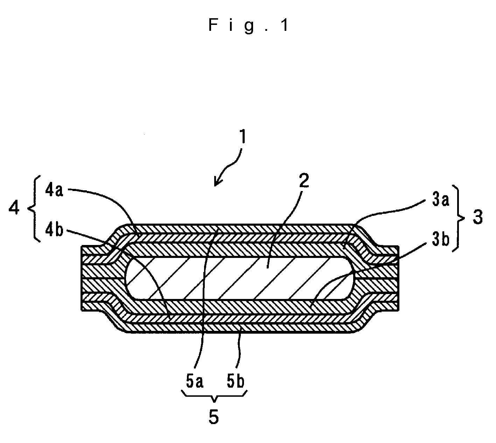

[Fig. 1] Fig. 1 is a schematic cross-sectional view showing a solid preparation in accordance with an embodiment of the present invention.

### DESCRIPTION OF EMBODIMENTS

The solid preparation of the present invention comprises a drug-containing unit containing a drug and a gel-forming layer for covering the drug-containing unit directly or indirectly and forming a gel by swelling with absorbing water and may comprise an intermediate layer interposed between the drug-containing unit and the gel-forming layer.

Fig. 1 shows a schematic cross-sectional view of a solid preparation in accordance with an embodiment of the present invention.

A solid preparation (an oral administration preparation) 1 shown in Fig. 1 comprises a drug-containing unit (or drug-containing layer) 2 containing a drug, an intermediate layer (or adhesive layer) 3 for covering the drug-containing unit, a gel-forming layer 4 for covering the intermediate layer and swelling with absorbing water to form a gel, and a water-soluble anti-adhesive layer (or outermost layer) 5 for covering the gel-forming layer and preventing adhesion inside the oral cavity (or buccal cavity). The drug-containing unit 2 contains a cationic drug, and the gel-forming layer 4 contains an anionic polymer. In this embodiment, the above-mentioned layers are laminated to form a laminate. That is, the intermediate layer 3 comprises a first intermediate layer 3a laminated on a first surface of the drug-containing unit 2 and a second intermediate layer 3b laminated on a second surface of the drug-containing unit 2. The first intermediate layer 3a and the second intermediate layer 3b are adhered (or bonded) together at the periphery of the drug-containing unit 2 to seal the drug-containing unit 2. Further, the gel-forming layer 4 comprises a first gel-forming layer 4a laminated on the first intermediate layer 3a and a second gel-forming layer 4b laminated on the second intermediate layer 3b. The anti-adhesive layer 5 comprises a first anti-adhesive layer 5a laminated on the first gel-forming layer 4a and a second anti-adhesive layer 5b laminated on the second gel-forming layer 4b.

Specifically, in this embodiment, the intermediate layer 3 is interposed between the drug-containing unit 2 containing and the cationic drug and the gel-forming layer 4 containing the anionic polymer, and contains an acidic component.

According to the solid preparation 1, the water-soluble anti-adhesive layer 5 is rapidly dissolved in water or moisture (e.g., saliva) in the oral cavity to form a lower-viscous layer (or membranous layer) on an outermost surface of the solid preparation. Thus, the gel-forming layer 4 can prevent adhesion of the solid preparation 1 to an inner wall of the oral cavity. Moreover, in the oral cavity, the gel-forming layer 4 absorbs saliva or water through the anti-adhesive layer 5 and swells to form a gel. Accordingly, even if a large quantity of water is absent, the solid preparation 1 changes into a smooth and slippery dosage form having easy-to-swallow size, shape, elasticity, viscosity, and other properties in the oral cavity, so that the solid preparation 1 can easily be administered (or given) to a patient. Moreover, the solid preparation 1 reduces a risk of blocking the respiratory tract of the patient, and thus the solid preparation 1 can safely be administered even to elderly people and infants.

Since the intermediate layer 3 contains the acidic component, the elution property of the drug can significantly be improved without adversely affecting the drug of the drug-containing unit 2. That is, when the cationic drug and the anionic polymer coexist, the drug is probably adsorbed (adsorbed due to ionic interaction) on or ionic-bonded to the anionic polymer. Accordingly, when the solid preparation 1 is subjected to a dissolution test, the elution property of the drug from the solid preparation 1 is significantly decreased even if the solid preparation 1 is disintegrated. In contrast, when the intermediate layer 3 contains the acidic component, the action of the anionic polymer on the drug of the drug-containing unit 2 can significantly be inhibited, and the adsorption or ionic bonding of the drug on or to the anionic polymer can effectively be prevented. Therefore, even when the cationic drug coexists with the anionic polymer, the elution property of the drug from the solid preparation can significantly be improved.

Incidentally, according to the solid preparation having the above-mentioned structure, the cationic drug and an acidic component may coexist in the drug-containing unit by incorporating the acidic component into the drug-containing unit. Even such a solid preparation has the same advantages as the solid preparation having the structure shown in Fig. 1. That is, even if the solid preparation contains the cationic drug and the anionic polymer, the elution property of the drug from the solid preparation can significantly be improved. Moreover, the anti-adhesive layer prevents the adhesion of the solid preparation to the inner wall of the oral cavity, and the gel-forming layer allows easy swallowing of the solid preparation while improving the comfortability (or feeling or acceptability) of taking the solid preparation.

Incidentally, the intermediate layer (or adhesive layer) is not necessarily required. In order to seal the drug-containing unit by bonding (or adhesively attaching) the first gel-forming layer to the second gel-forming layer, it is preferable that the solid preparation have the intermediate layer (or adhesive layer). Moreover, the anti-adhesive layer (surface layer) is not also necessarily required. The anti-adhesive layer can effectively prevent the adhesion of the solid preparation to the inner wall of the oral cavity and improve the comfortability of taking the solid preparation.

### [Drug-containing unit]

The cationic drug contained in the drug-containing unit (or drug-containing layer) has at least one basic group, for example, a primary amino group (-NH₂), a secondary amino group (imino group -NH-), a tertiary amino group (=N-), an amide group, a basic nitrogen-containing heterocyclic group (e.g., a pyrrolyl group, an imidazolyl group, a pyrazolyl group, a pyrazinyl group, a purinyl group, a quinolyl group, a pyridyl group, a piperidino group, a piperidyl group, a piperazinyl group, and a triazolo group). Incidentally, the amino group also includes a hydrazino group (-NH-NH₂), a hydrazo group (-NH-NH-), and others. It is sufficient that the cationic drug has at least one basic group, and the cationic drug may have a plurality of basic groups, whichmaybe the same or different in species. Moreover, the drug may form a salt [for example, a salt with an inorganic acid (e.g., hydrochloric acid, sulfuric acid, and phosphoric acid), an organic carboxylic acid (e.g., acetic acid, tartaric acid, citric acid, fumaric acid, and maleic acid), or an organic sulfonic acid (e.g., mesylic acid)].

The species of the cationic drug is not particularly limited to a specific one, and may for example be an antipyretic (or a febrifuge), an analgesic, an antiphlogistic (or an antiinflammatory agent), a hypnotic and a sedative, a rheumatism-treating agent (or an antirheumatic), an antivertigo agent, an antiallergic agent, a cardiant, a β-blocking agent, a calcium antagonist, an antiarrhythmic agent,a diuretic,an angina-treating agent, an agent for treating heart failure, an agent for treating myocardial infarction, a depressor (a hypertension-treating agent), an agent for treating disturbances of peripheral circulation, a vasopressor (a hypotension-treating agent), a bronchodilator, an antasthmatic, an antituberculous agent, a diabetic agent, an agent for treating diabetic complication, a hyperlithuria-treating agent, an antitussive expectorant, an agent for treating peptic ulcer, an agent for treating thyroid disease, a prostatomegaly-treating agent, a carcinostatic (or an anticancer agent), an osteoporosis-treating agent, an agent for treating Alzheimer's disease, an antibiotic, a vitamin compound, and an antiplasmin agent. The cationic drug is not limited to a pharmacologically active ingredient and may be a physiologically active ingredient.

Concrete examples of the cationic drug as a pharmacologically active ingredient may include a antipyretic, analgesic or antiphlogistic [e.g., an antipyretic analgesic (such as dimetotiazine mesilate), an anticephalalgic agent (such as dihydroergotamine mesilate, lomerizine hydrochloride, or sumatriptan succinate), and an antiphlogistic (such as fenamic acid, mefenamic acid, floctafenine, proglumetacin maleate, epirizole, or tiaramide hydrochloride)], an antirheumatic (such as penicillamine or methotrexate), a hyperlithuria-treating agent (such as allopurinol), a hypnotic and a sedative (such as rilmazafone hydrochloride or zolpidem tartrate), an antidepressant (such as nortriptyline hydrochloride, imipramine hydrochloride, amitriptyline hydrochloride, clomipramine hydrochloride, fluvoxamine maleate, or milnacipran hydrochloride), an antivertigo agent (such as isoprenaline hydrochloride or betahistine mesilate), an antiallergic agent (e.g., an antihistaminic agent such as diphenhydramine hydrochloride, diphenylpyraline teoclate, clemastine fumarate, chlorpheniramine maleate, alimemazine tartrate, or promethazine hydrochloride; and a histamine H₁ antagonist (or a basic antiallergic agent) such as ketotifen fumarate, azelastine hydrochloride, or epinastine hydrochloride), a cardiant (such as denopamine or isoprenaline hydrochloride), an antianginal agent (such as nicorandil, etafenone hydrochloride, dipyridamole, trapidil, or trimetazidine hydrochloride), a β-blocking agent (such as propranolol hydrochloride, difenidol hydrochloride, bufetolol hydrochloride, bupranolol hydrochloride, bopindolol malonate, oxprenolol hydrochloride, alprenolol hydrochloride, indenolol hydrochloride, acebutolol hydrochloride, or celiprolol hydrochloride), a calcium antagonist (such as manidipine hydrochloride, benidipine hydrochloride, amlodipine besilate, verapamil hydrochloride, or diltiazem hydrochloride), an antiarrhythmic agent (such as aprindine hydrochloride, pilsicainide hydrochloride, propafenone hydrochloride, aminodarone hydrochloride, nifekalant hydrochloride, sotalol hydrochloride, or bepridil hydrochloride), a diuretic (such as hydrochlorothiazide, penflutizide, benzylhydrochlorothiazide, bumetanide, azosemido, or triamterene), a depressor (e.g., a sympathetic blocking agent such as clonidine hydrochloride, methyldopa, guanabenz acetate, guanfacine hydrochloride, reserpine, prazosin hydrochloride, bunazosin hydrochloride, terazosin hydrochloride, or doxazosin mesilate; a vasodilator such as hydralazine hydrochloride, budralazine, todralazine hydrochloride, or cadralazine; an ACE inhibitor such as enalapril maleate, delapril hydrochloride, lisinopril, or benazepril hydrochloride; and angiotensin II receptor antagonist such as candesartan cilexetil or valsartan), an agent for treating disturbances of peripheral circulation (such asinositolhexanicotinate, hepronicate, tolazoline hydrochloride, or isoxsuprine hydrochloride), a vasopressor (such as metaraminol bitartrate, methoxamine hydrochloride, midodrine hydrochloride, amezinium metilsulfate, etilefrine hydrochloride, or phenylephrine hydrochloride), a bronchodilator and antasthmatic (e.g., a β₂-adrenergic receptor agonist such as ephedrine hydrochloride, methylephedrine hydrochloride, isoprenaline hydrochloride, orciprenaline sulfate, clorprenaline hydrochloride, salbutamol hydrochloride, terbutaline hydrochloride, formoterol fumarate, tulobuterol hydrochloride, fenoterol hydrobromide, procaterol hydrochloride, or clenbuterol hydrochloride; and a xanthine derivative such as theophylline, aminophylline, choline theophylline, or proxyphylline), an antitussive (such as dimemorfan phosphate, tipepidine hibenzate, oxeladin citrate, dextromethorphan hydrobromide, pentoxyverine citrate, chloperastine, or benproperine phosphate), a diabetic agent (such as tolbutamide, acetohexamide, glibenclamide, glimepiride, buformin hydrochloride, metformin hydrochloride, pioglitazone hydrochloride, or voglibose), an expectorant (such as L-methylcysteine hydrochloride, ambroxol hydrochloride, or bromhexine hydrochloride), an agent for treating peptic ulcer (e.g., an H₂ receptor antagonist such as cimetidine, ranitidine hydrochloride, or famotidine; a proton pump inhibitor such as lansoprazole or omeprazole; and a muscarine receptor antagonist such as pirenzepine hydrochloride), an antibiotic (such as clarithromycin, kitasamycin, josamycin, midecamycin, rokitamycin, or azithromycin), a narcotic (such as amphetamine or meperidine), a vitamin compound [e.g., a vitamin B₁ compound such as thiamine hydrochloride, thiamine nitrate, dicethiamine hydrochloride, cycotiamine, benfotiamine, bisibutiamine, fursultiamine, prosultiamine, octotiamine, bisbentiamine, or thiamine disulfide; a vitamin B₂ compound such as riboflavin, riboflavin sodium phosphate, riboflavin butyrate, or flavin adenine dinucleotide sodium; a vitamin B₆ compound such as pyridoxine hydrochloride, pyridoxine acetate, or pyridoxal phosphate; a nicotinic acid compound such as nicotinic acid, or nicotinamide; a vitamin B₁₂ compound such as mecobalamin, cyanocobalamin, hydroxocobalamin (such as hydroxocobalamin hydrochloride or hydroxocobalamin acetate), or methylcobalamin; folic acid, a pantothenic acid compound, biotin, and vitamin P (such as hesperidin)], and an antiplasmin agent (such as ε-aminocaproic acid or tranexamic acid). These pharmacologically active ingredients may be used alone or in combination.

As the cationic drug as a physiologically active ingredient, there may be mentioned an amino acid or a salt thereof [for example, glycine, L-lysine, L-valine, L-alanine, L-arginine, L-cystine, L-methionine, L-glutamic acid, and L-aspartic acid, or an alkali metal salt thereof (e.g., a sodium salt)], a peptide or a salt thereof [forexample, a peptide (such as L-lysineglutamate, or a collagen and a collagen peptide thereof), coenzyme Q₁₀, and L-carnitine or a salt thereof (such as a fumarate or tartrate)], a glucosamine compound (such as a chitin or a chitosan), and others. These physiologically active ingredients may be used alone or in combination. The physiologically active ingredient may be used in combination with the pharmacologically active ingredient.

Incidentally, the cationic drug may be used in combination with a neutral drug and/or an anionic drug [a drug having an anionic group (such as a carboxyl group or a sulfonic acid group)].

According to the present invention, since the drug-containing unit can be enclosed in the gel-forming layer, a physical strength can be imparted to the solid preparation even when the solid preparation contains a relatively large amount of an active ingredient, or a bulky active ingredient, which easily lowers the physical strength of the solid preparation. Thus, the present invention can be applied to both a slight or low dose (e.g., not more than 1 mg) of an active ingredient and a large or high dose (e.g., not less than 300 mg) of an active ingredient as the active ingredient. The unit dosage amount of the active ingredient may for example be about 0.01 to 1500 mg (e.g., about 0.01 to 800 mg), preferably about 0.1 to 1200 mg (e.g., about 0.1 to 500 mg), and more preferably about 1 to 1000 mg (e.g., about 1 to 300 mg) and is usually about 1 to 500 mg (e.g., about 2 to 250 mg). The active ingredient content can be selected according to the species of the active ingredient or others, and is usually, in the drug-containing unit, about 0.001 to 100% by mass, preferably about 0.01 to 70% by mass (e.g., about 0.01 to 50% by mass), and more preferably about 0.1 to 35% by mass.

The solid preparation of the present invention provides a comfortable feeling (or great ease) to take and can effectively be administered orally with a small quantity of water or substantially without water. Thus, for example, the solid preparation can suitably be used for an active ingredient having a large unit dosage amount, a bulky active ingredient, an unpalatable (such as bitter or acerbic) active ingredient, a highly water-soluble active ingredient. Among these ingredients, usually, the pharmacologically active ingredient is widely used.

The drug-containing unit may comprise the active ingredient alone, and usually contains an additive (a base material or a carrier). The additive is not particularly limited to a specific one, and depending on the shape of the preparation, a conventional carrier, for example, at least one carrier selected from the group consisting of an excipient, a binder, a disintegrant, and a lubricant may be selected.

As the excipient, there may be mentioned a saccharide such as lactose, white sugar or refined sugar, maltose, glucose, sucrose, or fructose (or fruit sugar); a sugar alcohol such as mannitol, sorbitol, or xylitol; a starch such as a corn starch or a potato starch; a polysaccharide such as a crystalline cellulose (including a microcrystalline cellulose), cyclodextrin, or dextran; silicon dioxide or a silicate such as a light silicic anhydride, a synthetic aluminum silicate, magnesium silicate, magnesium aluminometasilicate, or atalc; an oxide such as titanium oxide; a carbonate such as calcium carbonate or magnesium carbonate; a phosphate such as calcium monohydrogenphosphate; and others. The binder may include a water-soluble starch or starch derivative such as a pregelatinized starch, a partially pregelatinized starch, an oxidized starch, a sodium carboxymethyl starch, a hydroxypropyl starch, or dextrin; a polysaccharide such as agar, gum acacia (or gum arabic), dextrin, sodiumalginate, a tragacanth gum, a pullulan, a xanthan gum, a hyaluronic acid, a pectin, a sodium chondroitin sulfate, or a gelatin; a synthetic polymer such as a polyvinylpyrrolidone (e.g., a povidone), a vinyl acetate-vinylpyrrolidone copolymer, a poly(vinyl alcohol), a carboxyvinyl polymer, a polyacrylic acid-series polymer (orapolyacrylicpolymer), a polylactic acid, a poly(ethylene glycol), or a poly(vinyl acetate); a cellulose ether such as a methyl cellulose (MC), an ethyl cellulose (EC), a carboxymethyl cellulose (CMC), a carboxymethylethyl cellulose (CMEC), a hydroxypropyl cellulose (HPC), or a hydroxypropylmethyl cellulose (HPMC), and a cellulose ester such as a cellulose acetate; and others. The disintegrant may include calcium carbonate, a carboxymethyl cellulose or a salt thereof (e.g., a carmellose, a carmellose sodium, a carmellose calcium, and a croscarmellose sodium), a polyvinylpyrrolidone (e.g., a povidone and a crosslinked polyvinylpyrrolidone (crospovidone)), a low-substituted hydroxypropyl cellulose, magnesium aluminometasilicate, and others. The lubricant may include a talc, magnesium stearate, a poly(ethyleneglycol) 6000, and others. These carriers may be used alone or in combination.

The drug-containing unit may contain a polyglucosamine compound (such as a chitin or a chitosan), a protein (such as a casein or a soybean protein), an enteric base material (e.g., a cellulose derivative such as a cellulose phthalate, a cellulose acetate phthalate, a hydroxypropyl cellulose phthalate, a hydroxypropylmethyl cellulose phthalate (HPMCF), or a hydroxypropylmethyl acetate succinate, a methacrylic acid-ethyl acrylate copolymer (methacrylic acid copolymer LD), a methacrylic acid-n-butyl acrylate copolymer, and a methacrylic acid-methyl methacrylate copolymer (methacrylic acid copolymers L and S)), a gastric-soluble base material (a dimethylaminoethyl methacrylate-methacrylic acid copolymer, a dimethylaminoethyl methacrylate-methyl methacrylate copolymer, a dimethylaminoethyl methacrylate-chlorotrimethylammoniumethyl methacrylate copolymer, a dimethylaminoethyl methacrylate-chlorotrimethylammoniummethyl methacrylate copolymer, a dimethylaminoethyl methacrylate-chlorotrimethylammoniumethyl acrylate copolymer, a polyvinylacetal diethylaminoacetate), and others. Moreover, the enteric base material and/or gastric-soluble base material may be used as the binder.

Further, the drug-containing unit may contain a lipid. The lipid may include a wax (e.g., a bees wax, a carnauba wax, a cacao butter, a lanolin, a paraffin, and a petrolatum), a higher (or long chain) fatty acid ester [e.g., an alkyl ester of a saturated or unsaturated fatty acid, and an ester of a fatty acid with a polyhydric alcohol (such as a poly(C₂₋₄alkylene glycol), glycerin, or a polyglycerin) (e.g., a glyceride)], a hardened (or hydrogenated) oil, a higher alcohol (e.g., a saturated aliphatic alcohol such as stearyl alcohol and an unsaturated aliphatic alcohol such as oleyl alcohol), a higher fatty acid (e.g., linoleic acid, linolenic acid, oleic acid, and stearic acid), a metallic soap (e.g., a metal salt of a fatty acid, such as a sodium salt of palm oil fatty acid or calcium stearate), and others.

Furthermore, for the drug-containing unit, a known additive can be used. Such an additive may include, for example, a disintegrant aid (or adjuvant), an antioxidation agent or an antioxidant, a variety of surfactants such as a nonionic surfactant), a dispersing agent, an antiseptic agent or a preservative (e.g., a paraben such as methyl paraben or butyl paraben), a fungicide or antibacterial agent (e.g., a benzoic acid compound such as sodiumbenzoate), an antistatic agent, a corrigent or a masking agent (e.g., sweetening agent), a coloring agent (e.g., a dye and a pigment such as titanium oxide or colcothar), a deodorant or a flavoring agent (or perfume) (e.g., an aromatic substance), and an algefacient. These additives may be used alone or in combination.

The ratio of the additive may for example be about 0.001 to 100 parts by mass (e.g., about 0.01 to 50 parts by mass, preferably about 0.1 to 30 parts by mass, and more preferably about 0.5 to 20 parts by mass) relative to 1 part by mass of the active ingredient.

The drug-containing unit containing the active ingredient and the additive (base material or carrier) may be shaped or formed into various shapes or dosage forms of solid preparations, for example, powdered preparations, powders, granulated preparations (e.g., granules and microfine granules), spherical or spheroidal preparations, tablets (including sublingual tablets, orally disintegrating tablets, troches, chewable tablets, and others), capsules (including hard capsules, soft capsules, and microcapsules), and layered or film-covered preparations (or sheet-shaped preparations). The shape (or form) of the drug-containing unit may for example be a spherical shape, an ellipsoidal shape, a polyhedral or prismatic shape, a layered shape, an amorphous shape, and an aggregate of particles. Incidentally, granulation or covering of the cationic drug with the additive (base material or carrier) in the form of granules or the like can prevent the cationic drug from contacting with the component (s) of the adjacent layer and improve the stability of the cationic drug in some cases.

According to the present invention, even when the solid preparation has a large contact surface area with the inner wall of the oral cavity due to the shape of the preparation, the solid preparation can easily be swallowed without water or with a small quantity of water. Moreover, the preparation can easily be swallowed even in spite of a high drug content and a large dosage size. Thus, the drug-containing unit may be formed as a preparation that is conventionally difficult for elderly people and infants (babies and little children) to swallow [for example, a preparation having a flat region or plateau, a preparation having a flat shape, and a large-sized tablet (e.g., a tablet having a diameter of about 5 to 15 mm, preferably about 6 to 14 mm, and more preferably about 7 to 13 mm)]. Among these shapes, the drug-containing unit may have a layered or film-like shape (e.g., a polygon such as a quadrilateral, a circle, and an ellipse). The layered drug-containing unit may for example have a thickness of about 5 µm to 5 mm, preferably about 10 µm to 3 mm, and more preferably about 100 to 1000 µm (e.g., about 100 to 500 µm).

### [Intermediate layer]

When the drug-containing unit contains the after-mentioned pharmaceutically acceptable acidic component, an intermediate layer (or adhesive layer) is not necessarily required between the drug-containing unit and the gel-forming layer. However, when the first and second gel-forming layers are joined (or adhered) together through the intermediate layer (or adhesive layer) at the periphery of the drug-containing unit, the intermediate layerintimatelyjoins (or adheres) these gel-forming layers to each other, effectively prevents leakage of the active ingredient from the drug-containing unit, and allows smooth administration of the preparation.

On the other hand, when the drug-containing unit does not contain the pharmaceutically acceptable acidic component, incorporation of the pharmaceutically acceptable acidic component into the intermediate layer can improve the elution property of the drug.

Incorporation of the pharmaceutically acceptable acidic component into both the drug-containing unit and the intermediate layer can further improve the elution property of the drug.

The base material (adhesive) of the intermediate layer (or adhesive layer) may be either a water-soluble adhesive or a water-insoluble adhesive. As the water-soluble adhesive, there may be mentioned a (meth)acrylic acid-series polymer (or a (meth)acrylic polymer) [for example, a polyacrylic acid or a salt thereof (such as a carboxyvinyl polymer or a poly (sodium acrylate)); and an acrylic acid copolymer or a salt thereof], a vinylpyrrolidone-series polymer [a povidone, and a copolymer of vinylpyrrolidone such as a vinyl acetate-vinylpyrrolidone copolymer], a polysaccharide [for example, a polysaccharide derived from a plant (e.g., a cellulose derivative such as a CMC, a CMC sodium salt, an MC, an HPC, or an HPMC, a karaya gum, a pectin, a guar gum, a locust bean gum, a gum acacia (or gum arabic), a tragacanth gum, a carrageenan, and alginic acid or a sodium salt thereof), and a polysaccharide derived from a fungus (e.g., an acidic polysaccharide such as a pullulan, a xanthan gum, a hyaluronic acid, or a chondroitin sulfate or a sodium salt thereof)], and others. Examples of the water-insoluble adhesive (for example, an adhesive soluble in an organic solvent such as ethanol or acetone) may include a vinyl acetate-series polymer (e.g., a poly((vinyl acetate) and an ethylene-vinyl acetate copolymer), a (meth) acrylic acid-series polymer [e.g., a methacrylic acid-ethyl acrylate copolymer (methacrylic acid copolymer LD), a methacrylic acid-n-butyl acrylate copolymer, and a methacrylic acid-methyl methacrylate copolymer (methacrylic acid copolymers L and S)], and others. The water-soluble (meth)acrylic acid-series polymer may include the same polymer as the after-mentioned gel-forming agent or anionic polymer for the anti-adhesive layer. These adhesives may be used alone or in combination.

The adhesive may have heat (or thermal) adhesiveness (hot-melting property). Such an adhesive having heat adhesiveness may include a (meth)acrylic acid-series polymer, a vinylpyrrolidone-series polymer, a vinyl acetate-series polymer, and others.

As the adhesive, a water-soluble polymer is practically used, and there may be mentioned a (meth) acrylic acid-series polymer (such as a carboxyvinyl polymer) and a vinylpyrrolidone-series polymer (such as a povidone) as the water-soluble polymer. Moreover, when an adhesive having both water solubility and heat adhesiveness is used, the drug-containing unit can be sealed in a simple operation by interposing the drug-containing unit between a pair of film-like adhesive layers and heat-adhering (heat-bonding) the adhesive layers each other at the periphery of the drug-containing unit.

The adhesive layer may contain a plasticizer. Examples of the plasticizer may include a water-soluble plasticizer [e.g., ethylene glycol, propylene glycol, glycerin, sorbitol, sucrose, a polyoxyethylene polyoxypropylene glycol (such as pluronic or poloxamer), a polyoxyethylene sorbitan fatty acid ester (such as polysorbate 80), and a poly(ethylene glycol) (such as macrogol 400, 600, 1500, 4000, or 6000)], a water-insoluble plasticizer (e.g., triacetin, triethyl citrate, diethyl phthalate, dioctyl adipate, and a fatty acid such as lauric acid), and others. These plasticizers may be used alone or in combination. The preferred plasticizer includes a water-soluble plasticizer, such as glycerin.

The amount of the plasticizer may be selected according to the species of the base material (adhesive) of the adhesive layer, and may be about 1 to 100 parts by mass, preferably about 5 to 75 parts by mass (e.g., about 10 to 50 parts by mass), and more preferably about 15 to 50 parts bymass (e.g., about 20 to 40 parts bymass) relative to 100 parts by mass of the base material.

The adhesive layer may cover (or coat) at least part of the surface of the drug-containing unit to adhere (or bond) the drug-containing unit to the gel-forming layer. The adhesive layer may usually cover (or coat) the whole or part of the surface of the drug-containing unit (for example, at least upper and under surfaces of a layered drug-containing unit).

The thickness of the adhesive layer may be selected from a wide range of, for example, about 1 µm to 1 mm (e.g., about 5 to 500 µm) as far as the drug-containing unit is not exposed. The thickness of the adhesive layer may be about 10 to 500 µm (e.g., about 15 to 300 µm), preferably about 20 to 200 µm (e.g., about 30 to 175 µm), and more preferably about 50 to 150 µm.

### [Gel-forming layer]

The gel-forming layer contains an anionic or acidic polymer as a gel-forming agent. The gel-forming layer encloses (or wraps) the drug-containing unit and gelates by a small quantity of water such as saliva, so that the gel-forming layer changes a shape or surface characteristic of the preparation to impart a significantly improved slipperiness and an elasticity or viscosity suitable for easy swallowing to the preparation. Thus the comfortability (or feeling) of taking the preparation is improved (for example, the gel-forming layer facilitates the swallowing of the preparation).

It is sufficient that the gel-forming agent of the gel-forming layer contains at least a pharmaceutically acceptable anionic or acidic polymer which may be a synthetic polymer, a cellulose derivative, a starch derivative, a natural polysaccharide, and others. The anionic or acidic polymer for the gel-forming agent may include a carboxyl group-containing polymer (or macromolecule) [a synthetic polymer such as a carboxyl group-containing polymer obtainable from at least one polymerizable monomer selected from the group consisting of (meth) acrylic acid and itaconic acid as a polymerizable component, or a carboxyvinyl polymer; a cellulose derivative such as a CMC, a carboxymethylethyl cellulose, or a carboxymethylhydroxyethyl cellulose; a starch derivative such as a carboxymethyl starch; and a natural polysaccharide such as alginic acid, a heparin, a hyaluronic acid, a pectin, a tragacanth gum, a xanthan gum, or a gellan gum], a sulfonic acid group-containing polymer [e.g., a synthetic polymer such as a poly(styrene sulfonic acid), a poly(ethylene sulfonic acid), a poly(vinyl sulfate), a cellulose derivative such as a cellulose sulfate, and a natural polysaccharide such as a hyaluronic acid, a carrageenan, or a chondroitin sulfate], a phosphoric acid group-containing polymer (e.g., a cellulose derivative such as a cellulose phosphate), or a salt thereof, and others. These anionic or acidic polymers may be used alone or in combination.

The anionic polymer may form, for example, a salt with an inorganic base [an alkali metal (such as sodium or potassium), ammonia] or an organic base [such as monoethanolamine, diethanolamine, triethanolamine, or dimethylaminoethanol].

Among these anionic polymers, in order to absorb water or moisture rapidly, it is preferred to use a water-soluble anionic polymer, for example, a carboxy group-containing polymer and a sulfonic acid group-containing polymer, particularly an anionic polymer comprising a (meth)acrylic acid unit as an essential polymerizable component (a homo- or copolymer of (meth) acrylic acid, or a (meth)acrylicacid-seriespolymer). As a monomer copolymerized with (meth)acrylic acid (copolymerizable monomer), there may be mentioned an alkyl (meth)acrylate [for example, a C₁₋₆alkyl (meth)acrylate such as methyl (meth)acrylate, ethyl (meth)acrylate, or butyl (meth)acrylate, particularly a C₁₋₄alkyl (meth)acrylate], a hydroxyalkyl (meth)acrylate [for example, a hydroxyC₂₋₄alkyl (meth)acrylate such as hydroxyethyl (meth)acrylate or hydroxypropyl (meth)acrylate, particularly a hydroxyC₂₋₃alkyl (meth)acrylate], vinyl acetate, vinylpyrrolidone, and others. These copolymerizable monomers may be used alone or in combination.

The mass ratio of the (meth) acrylic acid relative to the copolymerizable monomer may for example be about 100/0 to 50/50, preferably about 100/0 to 60/40 (e.g., about 99.9/0.1 to 65/35), and more preferably about 100/0 to 70/30 (e.g., about 99/1 to 80/20), as the (meth) acrylic acid/the copolymerizable monomer.

The (meth) acrylic acid-series polymer may include a poly((meth)acrylic acid), a (meth)acrylic acid-methyl (meth)acrylate copolymer, a (meth)acrylic acid-ethyl (meth)acrylate copolymer, a (meth)acrylic acid-butyl (meth) acrylate copolymer, and others. These (meth) acrylic acid-series polymers may be used alone or in combination.

Representative examples of the (meth)acrylic acid-series polymer includes a carboxyvinyl polymer (trade name: CARBOPOL), a poly(sodium acrylate), a partially neutralized product of a polyacrylic acid, a methacrylic acid-n-butyl acrylate copolymer, and a methacrylic acid copolymer LD (trade name: EUDRAGIT L-30D55). Among these (meth) acrylic acid-series polymers, a polyacrylic acid or an acrylic acid copolymer in each of which acrylic acid as a main monomer is polymerized (that is, an acrylic acid-series polymer), particularly a carboxyvinyl polymer, is preferred. As the carboxyvinyl polymer, there may be mentioned CARBOPOL 981, CARBOPOL 980, CARBOPOL 974P, CARBOPOL 971P, CARBOPOL 941, CARBOPOL 940, CARBOPOL 934P, CARBOPOL 71G (manufactured by Noveon, US), HIVISWAKO 103, HIVISWAKO 104 (manufactured by Wako Pure Chemical Industries, Ltd.), JUNLON (Nihon Junyaku Co., Ltd.), AQUPEC (Sumitomo Seika Chemicals Company Limited), and others.

The anionic polymer (e.g., a carboxyvinyl polymer) may have a Viscosity of about 1500 to 50000 mPa·s, preferably about 2500 to 20000 mPa·s, more preferably about 5000 to 15000 mPa·s, and particularly about 7500 to 12500 mPa·s (e.g., 8000 to 12000 mPa·s) for a 0.2% by mass aqueous solution at 20°C.

Incidentally, if necessary, the anionic polymer may be used in combination with other gel-forming agents, for example, a protein (such as a collagen or a casein), a hydroxyl group-containing polymer (e.g., a synthetic polymer such as a poly(vinyl alcohol), a cellulose derivative such as an MC, a HPC, or an HPMC, a starch derivative such as a hydroxypropyl starch or a dextrin, and a natural polysaccharide such as an agar, a galactomannan, a glucomannan, a guar gum, a locust bean gum, a gum acacia (or gum arabic), an arabinogalactan, a tamarind gum, a psyllium seed gum, or a dextran).

The anionic polymer content of the gel-forming layer may be selected from a range in which the anionic polymer can absorb water rapidly to form a gel and inhibit the dissolution of the gel-forming agent and may for example be about 5 to 90% by mass (e.g., about 10 to 80% by mass) in terms of a non-volatile matter. The anionic polymer content of the gel-forming layer may be about 10 to 70% by mass (e.g., about 12 to 50% by mass) and preferably about 15 to 35% by mass (e.g., about 15 to 25% by mass) in terms of a non-volatile matter relative to the whole gel-forming layer.

The gel-forming layer may contain a pharmaceutically acceptable base material or a film-forming agent. The base material (film-forming agent) inhibits cracks of the gel-forming layer, stabilizes the shape of the gel-forming layer, and prevents the separation of the gel from the drug-containing unit.

Examples of the base material (film-forming agent) may include a vinyl-series polymer [for example, a (meth) acrylicpolymer, a vinyl alcohol-seriespolymer (such as a poly(vinyl alcohol)), a vinylpyrrolidone-series polymer (such as a povidone or a vinyl acetate-vinylpyrrolidone copolymer), a poly(vinyl acetate), and a poly(vinyl acetate phthalate)], a poly(ethylene glycol), and a polysaccharide derived from a plant [for example, a cellulose ether (e.g., an MC, a hydroxymethyl cellulose (HMC), an HEC, an HPC, and an HPMC), a xanthan gum, and a carrageenan]. These components may be used alone or in combination.

Among these film-forming agents, a water-soluble base material [for example, a poly(vinyl alcohol), a vinylpyrrolidone-series polymer, and a cellulose ether] is preferred. Use of the water-soluble base material facilitates the permeation (or infiltration) of water in the gel-forming layer, and the gel-forming layer can rapidly swell in the oral cavity to form a gel. In particular, use of the vinyl alcohol-series polymer (e.g., a poly(vinyl alcohol)) is useful for shielding and masking unpleasant taste and smell of the active ingredient contained in the drug-containing unit.

The base material content of the whole gel-forming layer may be selected from the range of about 20 to 85% by mass (e.g., about 30 to 80% by mass) and may usually be about 50 to 85% by mass and preferably about 60 to 80% by mass (e.g., about 65 to 75% by mass).

The mass ratio of the base material (film-forming agent) relative to the gel-forming agent (e.g., an anionic polymer) may be selected from the range of about 99/1 to 10/90 (e.g., about 90/10 to 15/85, particularly about 85/15 to 20/80) in terms of a solid content, and may usually be about 85/15 to 50/50 (e.g., about 82.5/17.5 to 65/35) and preferably about 80/20 to 70/30, as the base material/the gel-forming agent. The ratio of the base material relative to 100 parts by mass of the gel-forming agent may for example be about 50 to 700 parts by mass (e.g., about 100 to 500 parts by mass), preferably about 200 to 400 parts by mass, and more preferably about 250 to 350 parts by mass.

The gel-forming layer can for example be formed as a crosslinked gel-forming layer obtainable from a composition containing the gel-forming agent and a crosslinking agent. The crosslinked gel layer can form a gel having a high strength even in swelling due to water absorption, and having an elasticity and a high slipperiness in the oral cavity. Such a gel facilitates swallowing of the solid preparation and prevents dissolution in the oral cavity.

As the crosslinking agent for the anionic polymer, for example, a polyvalent metal compound can be used. The polyvalent metal compound is not particularly limited to a specific one as far as the compound is a pharmaceutically acceptable metal compound. Such a metal compound may include, for example, a polyvalent metal salt, a polyvalent metal oxide, a polyvalent metal hydroxide, and a polyvalent metal carbonate. Examples of the polyvalent metal may include an alkaline earth metal [for example, magnesium and calcium], and metals of the groups 3 to 13 of the Periodic Table of Elements [for example, a metal of the group 8 of the Periodic Table of Elements (e.g., iron), a metal of the group 12 of the Periodic Table of Elements (e.g., zinc), and a metal of the group 13 of the Periodic Table of Elements (e.g., aluminum)].

As these polyvalent metal compounds, for example, there may be mentioned calcium oxide, calcium chloride, magnesium oxide, magnesium chloride, zinc oxide, zinc sulfate, ferric sulfate, iron citrate, aluminum chloride, aluminum hydroxide, aluminum sulfate, aluminum silicate, aluminum phosphate, and an alum compound (for example, aluminum potassium sulfate (potassium alum), ammonium ion (III) sulfate dodecahydrate (ammonium iron alum), and aluminum ammonium sulfate (ammonium alum)). These polyvalent metal compounds may be used alone or in combination. Incidentally, use of a trivalent metal compound increases the degree of crosslinking of the gel-forming agent to improve the physical strength of the gel-forming layer and to prevent the dissolution of the gel-forming agent certainly (or surely).

Regarding the ratio (mass ratio) of the gel-forming agent (e.g., an anionic polymer) relative to the crosslinking agent, the ratio of the crosslinking agent relative to 100 parts by mass of the gel-forming agent is, for example, about 0.1 to 10 parts by mass (e.g., about 0.5 to 7.5 parts by mass), preferably about 1 to 5 parts by mass, and more preferably about 1.5 to 3.5 parts by mass (e.g., about 2 to 3 parts by mass). The crosslinking of the gel-forming agent with the crosslinking agent can retain the form (or shape) of the gel-forming layer while preventing the dissolution of the gel-forming layer. Moreover, a viscosity of a liquid coating composition as a material of the gel-forming layer can be lowered by regulating the ratio of the gel-forming agent and the crosslinking agent to form the gel-forming layer further efficiently.

Further, the ratio of the crosslinking agent relative to 100 parts by mass of the total amount of the base material and the gel-forming agent (e.g., an anionic polymer) may for example be about 0.1 to 2.5 parts by mass, preferably about 0.2 to 1.5 parts by mass (e.g., about 0.25 to 1.2 parts by mass), and more preferably about 0.3 to 1 parts by mass (e.g., about 0.5 to 0.8 parts by mass).

In order to increase the water-absorption speed and gelation speed, the gel-forming layer may contain a water absorption promoter. As the water absorption promoter, there may be used a highly water-soluble component. Examples of the water absorption promoter may include a monosaccharide or a disaccharide (for example, glucose, xylose, mannose, fructose, galactose, sucrose, fruit sugar (or levulose), and white sugar or refined sugar), a polyhydric alcohol [for example, an alkanediol (e.g., propylene glycol), a poly(ethylene glycol) (e.g., a poly(ethylene glycol) such as macrogol 300, macrogol 400, macrogol 600, macrogol 1500, macrogol 4000, or macrogol 20000; and a polyoxyethylene polyoxypropylene glycol), and a polyol having three or more hydroxyl groups (a tri- to polyvalent polyol) (e.g., glycerin), a sugar alcohol (e.g., erythritol, sorbitol, xylitol, mannitol, inositol, maltitol, and lactitol)], and an ethylene oxide adduct (e.g., polyoxyl 40 stearate, polyoxyl 45 stearate, polyoxyl 55 stearate, and polyoxyethylene hydrogenated castor oil). These water absorption promoters may be used alone or in combination.

Among these water absorption promoters, the polyhydric alcohol, particularly glycerin, is preferred, since the polyhydric alcohol has an excellent ability to accelerate water absorption and imparts flexibility to the gel to further ease swallowing of the solid preparation. Moreover, the monosaccharide or the disaccharide, the sugar alcohol or the glycerin can also mask the bitterness, acerbity and other unpleasant tastes of the drug.

The water absorption promoter may have a viscosity of about 0.3 to 5.0 mPa·s, preferably about 0.5 to 3.5 mPa·s, and more preferably about 0.6 to 2.5 mPa·s (e.g., about 0.6 to 2 mPa·s) for a 5% by mass aqueous solution at 37°C, and may have a viscosity of about 0.6 to 1.8 mPa·s for a 5% by mass aqueous solution at 37°C. The lower the viscosity of the aqueous solution of the water absorption promoter is, the higher the water-absorption speed of the gel-forming layer is.

From the point of view of form (or shape) retention and water absorption (percentage of water absorption) of the gel, the mass ratio of the water absorption promoter relative to 100 parts by mass of the gel-forming agent may be about 1 to 100 parts by mass, preferably about 5 to 75 parts by mass, and more preferably about 10 to 50 parts by mass (e.g., about 25 to 50 parts by mass). Incidentally, when a plurality of water absorption promoters containing glycerin is used, the glycerin content of the whole water absorption promoter may be about 35 to 95% by mass and preferably about 40 to 90% by mass.

The gel-forming layer may contain various optional components, for example, a plasticizer, a masking agent, an antiseptic agent, and a coloring agent, as with the after-mentioned anti-adhesive layer.

It is sufficient that the gel-forming layer covers at least part of the surface of the drug-containing unit (or the surface of the adhesive layer when the solid preparation contains the adhesive layer), particularly, the whole or the most of the surface thereof (for example, about 50 to 100% and preferably about 80 to 100%). The gel-forming layer may cover the surface area of the drug-containing unit or that of the adhesive layer uniformly or nonuniformly (scatteringly in a polygonal pattern such as quadrilateral pattern, a circular pattern, or a grid pattern). The gel-forming layer usually covers the whole of the drug-containing unit or that the adhesive layer (in the above-mentioned embodiment, at least upper and under surfaces).

The thickness of the gel-forming layer may be selected from the range of, for example, about 1 to 1000 µm (e.g., about 3 to 700 µm) and may be about 5 to 500 µm, and preferably about 7 to 250 µm (e.g., about 10 to 100 µm). Even a layer having a thickness of about 5 to 50 µm (e.g., about 10 to 30 µm) performs a sufficient function as the gel-forming layer. Incidentally, when the gel-forming layer is prepared, a plurality of thin gel-forming layers [gel-forming layers, each having a thickness of not more than 10 µm (e.g., about 1 to 10 µm, preferably about 2 to 9 µm, and more preferably about 3 to 8 µm)] may be laminated (or layered) to form a gel-forming layer having a predetermined thickness, thereby accelerating the gelation speed, according to a method described in Japanese Patent Application Laid-Open No. 2008-37794.

### [Pharmaceutically acceptable acidic component]

The pharmaceutically acceptable acidic component may be incorporated into the drug-containing unit and/or the intermediate layer, and if necessary, may be incorporated into the gel-forming layer and/or the anti-adhesive layer. Moreover, if necessary, the acidic component may be used as an acidifier (or an acidulant). Incidentally, when the acidic component is incorporated into the intermediate layer, it is advantageous in view of the stability and others of the preparation due to a lower possibility of adverse affects on the drug.

The pharmaceutically acceptable acidic component is not particularly limited to a specific one as far as the adsorption or bonding of the cationic drug on or to the anionic polymer can be prevented. As the acidic component, a pharmaceutically acceptable acid (or an edible acid) is usually employed. Moreover, the species of the acidic component may be selected according to the basicity of the drug, the acidity of the anionic polymer, and others. The acidic component practically has an acid dissociation constant pKa less than the acid dissociation constant pKa of acrylic acid (4.26). The acidic component may be either an inorganic acid or an organic acid. The acid dissociation constant pKa of the acidic component is usually not more than 4 (e.g., about -8 to 4.0), preferably about -8 to 3.8 (e.g., about 1 to 3.5), more preferably about 1.2 to 3.5, and particularly about 1.5 to 3.3. Incidentally, for an acidic component which dissociates in multistep, the value of the acid dissociation constant pKa sometimes varies depending on the number of dissociation steps. In this description, it is sufficient that the acid dissociation constant pKa in at least the first step (the number of dissociation steps: 1) or the second step (the number of dissociation steps: 2) is a predetermined value. Moreover, the pKa of the acidic component may be referred to Handbook of Chemistry, Basic Part II revised third edition (edited by The Chemical Society of Japan, published by Maruzen Company, Limited). Hereinafter, concrete examples of the acid are mentioned, and the numeral in the parenthesis indicates an acid dissociation constant pKa for reference.

As the inorganic acid, there may be mentioned, for example, hydrochloric acid (-8), HNO₂ (3.15), phosphoric acid (H₃PO₄, 2.15), diphosphoric acid (0.8), tripolyphosphoric acid (H₅P₃O₁₀, 2.0), sulfuric acid (1.99), and phosphonic acid (1.5).

The organic acid may include, for example, an aliphatic polyvalent carboxylic acid [e.g., a saturated dicarboxylic acid (such as oxalic acid (1.04), malonic acid (2.65), or succinic acid (4.00)) and an unsaturated dicarboxylic acid (such as maleic acid (1.75) or fumaric acid (2.85))], a hydroxycarboxylic acid [e.g., glycolic acid (3.63), lactic acid (3.66), malic acid (3.24), tartaric acid (2.82-2.99), citric acid (2.87), and gluconic acid (3.86)], an aldehydic acid or a ketonic acid [e.g., glyoxylic acid (3.18) and pyruvic acid (2.26)], an aromatic monocarboxylic acid [e.g., a benzoic acid (such as aminobenzoic acid (2.02-3.12)) and salicylic acid (2.81)], a heterocyclic monocarboxylic acid [e.g., nicotinic acid (2.05)], a heterocyclic polyvalent carboxylic acid [e.g., a pyridinedicarboxylic acid such as 2, 6-pyridinedicarboxylic acid (2.09)], and a sulfonic acid [e.g., mesylic acid (methanesulfonic acid, 0-2), ethanesulfonic acid, tosylic acid (-2.8), and benzenesulfonic acid (-2.5)].

The organic acid may be an amino acid compound [an amino acid or an amino acid derivative (for example, an amino acid having a substituent, and a peptide comprising 2 to 5 amino acid units)]. The amino acid compound may include, for example, an amino acid [e.g., asparagine (2.14), aspartic acid (1.93), alanine (2.30), arginine (2.05), isoleucine (2.32), glycine (2.36), glutamine (2.17), glutamic acid (2.18), serine (2.13), tyrosine (2.17), tryptophan (2.35), threonine (2.21), norleucine (2.30), valine (2.26), phenylalanine (2.26), methionine (2.15), lysine (2.04), and leucine (2.35)], an amino acid derivative [e.g., adenosine (3.50), L-alanyl-L-alanine (3.20), L-alanylglycine (3.10), L-alanylglycylglycylglycine (3.18), glycyl-L-alanine(3.07), glycylglycyl-L-alanine (3.18), glycylglycylglycylglycine (3.18), glycylglycyl-L-histidine (2.72), glycylglycylglycyl-L-histidine (2.90), glycyl-DL-histidylglycine (3.26), glycyl-L-histidine (2.54), N-methylglycine (sarcosine, 2.20), N,N-dimethylglycine (2.08), citrulline (2.43), and L-histidylglycine (2.84).

These acidic components may be used alone or in combination. The practically used acidic component includes an aliphatic polyvalent carboxylic acid [e.g., a saturated or unsaturated C₂₋₄dicarboxylic acid such as malonic acid, succinic acid, glutaric acid, maleic acid, or fumaric acid], a hydroxycarboxylic acid [e.g., a C₁₋₆hydroxycarboxylic acid such as lactic acid, malic acid, tartaric acid, citric acid, or gluconic acid], and an amino acid compound [an amino acid or a derivative thereof]. In particular, as a highly safe acidic component (e.g., an edible acid), at least one member selected from succinic acid, maleic acid, fumaric acid, lactic acid, malic acid, tartaric acid, citric acid, and gluconic acid is used practically. These acids may be water-insoluble, and an advantageous acid is water-soluble.

The acidic component may be used as a free acid, or an alkali metal salt or an alkaline earth metal salt. Examples of the alkali metal may include lithium, potassium, and sodium. Examples of the alkaline earth metal may include calcium and magnesium.

Considering from the easy water-solubility, the molecular weight of the acidic component is not more than 1000 g/mol and preferably about 90 to 500 g/mol.

The amount of the acidic component may be selected from the range preventing the adsorption of the cationic drug to the anionic polymer, and for example, may be about 1 to 5000 parts by mass (e.g., about 10 to 3000 parts by mass), preferably about 25 to 2500 parts by mass (e.g., about 50 to 2000 parts by mass), and more preferably about 75 to 1700 parts by mass (e.g., about 100 to 1500 parts by mass) relative to 100 parts by mass of the cationic drug.

### [Anti-adhesive layer (surface layer)]

The anti-adhesive layer (surface layer) is not necessarily required, and covering of the gel-forming layer with the anti-adhesive layer (surface layer) directly or indirectly is advantageous to prevention of adhesion of the solid preparation to the inner wall of the oral cavity by dissolving the anti-adhesive layer in water. Therefore, the preparation provided with the anti-adhesive layer (surface layer) covering the gel-forming layer is useful for extensive improvement of medication compliance from infants to elderly people.

As the component of the anti-adhesive layer (surface layer), for example, there may be mentioned a water-soluble polymer [e.g., a cellulose derivative [an alkyl cellulose (such as an MC), a hydroxyalkyl cellulose (such as an HEC, an HPC, or an HPMC), and a carboxymethyl cellulose (such as a CMC or a CMC-sodium)], a poly(ethylene glycol), a polyoxyethylene polyoxypropylene glycol, a poly(vinyl alcohol), an ethylene adduct of a higher fatty acid or polyhydric alcoholfatty acidester(e.g.,a polyoxyethylene stearate, a polyoxyethylene sucrose fatty acid ester, a polyoxyethylene sorbitan fatty acid ester, and a polyoxyethylene hydrogenated castor oil), a natural polysaccharide (such as a gum acacia (or gum arable)), and a protein (such as a gelatin)]; a saccharide [e.g., erythritol, sorbitol, xylitol, mannitol, inositol, maltitol, lactitol, glucose, xylose, mannose, fructose, galactose, lactose, white sugar or refined sugar, maltose, glucose, sucrose, and fruit sugar (or levulose)]; and a polyhydric alcohol (e.g., propylene glycol and glycerin). These components may be used alone or in combination. Among these components, a water-soluble polymer [for example, a cellulose derivative (e.g., an alkyl cellulose (such as an MC) and a hydroxyalkyl cellulose (such as an HEC, an HPC, or an HPMC)), a poly (ethylene glycol), are a poly (vinyl alcohol)] is practically used.

The preferred anti-adhesive layer (surface layer) contains a water-soluble cellulose ether and an anionic polymer and prevents adhesion of the solid preparation to the inner wall of the oral cavity. Such an anti-adhesive layer is dissolved by a small quantity of water or moisture (e.g., saliva) and more certainly forms an aqueous liquid coat around the gel formed from the gel-forming layer due to water absorption and swelling. Accordingly, the direct adhesion (attachment) of the gel-forming layer to the inner wall of the oral cavity can be prevented, and even if part of the gel-forming layer is adhered, the gel-forming layer is easily separated from the inner wall. Moreover, for oral administration, the adhesion of the solid preparation to the inner wall of the oral cavity over a longer period of time can certainly be prevented.

The water-soluble cellulose ether may include an alkyl cellulose [for example, a methyl cellulose (MC)], a hydroxyalkyl cellulose [for example, a hydroxyethyl cellulose (HEC) and a hydroxypropyl cellulose (HPC)], and a hydroxyalkylalkyl cellulose [for example, a hydroxyethylmethyl cellulose (HEMC) and a hydroxypropylmethyl cellulose (HPMC) (e.g., HPMC2208, HPMC2906, and HPMC2910)], a carboxymethyl cellulose [e.g., a carboxymethyl cellulose (CMC) and a CMC-sodium], and others. These cellulose ethers may be used alone or in combination.

Among these water-soluble cellulose ethers, the preferred one includes at least one member selected from the group consisting of a methyl cellulose, a hydroxyethyl cellulose, a hydroxyethylmethyl cellulose, a hydroxypropyl cellulose, and a hydroxypropylmethyl cellulose. Incidentally, for the water-soluble cellulose ether, the hydroxyalkyl cellulose (e.g., an HEC and an HPC), the hydroxyalkylalkyl cellulose (e.g., a hydroxyC₂-₃alkylmethyl cellulose such as an HEMC or anHPMC), and the alkyl cellulose (e.g., anMC) seems to have an action preventing the adhesion of the solid preparation to the inner wall of the oral cavity in descending order of degree.

In the hydroxyalkylmethyl cellulose, the content of ether groups derived from all hydroxyl groups of the cellulose is not particularly limited to a specific one. In order to prevent the adhesion of the solid preparation to the inner wall of the oral cavity, it is preferable that the average substitution degree of methyl group be larger and the average substitution degree of hydroxyalkyl group be smaller. Concretely, the methoxy group content (substitution ratio) may for example be about 5 to 40%, preferably about 10 to 35%, and more preferably about 15 to 30%; and the hydroxyalkoxy group content (substitution ratio) may for example be about 0.1 to 20%, preferably about 1 to 15%, and more preferably about 2 to 10%. The ratio of the methoxy group content (substitution ratio) relative to the hydroxyalkoxy group content (substitution ratio) may for example be about 90/10 to 50/50, preferably about 85/15 to 60/40, and more preferably about 80/20 to 70/30, as the methoxy group/the hydroxyalkoxy group.

Among the hydroxyalkylmethyl celluloses, an HPMC is preferred. Representative examples of the HPMC may include HPMC2208, HPMC2906, and HPMC2910, and HPMC2910 is particularly preferred.

The viscosity of the water-soluble cellulose ether for a 2% by mass aqueous solution at 20°C may be not more than 50 mPa·s, preferably not more than 40 mPa·s, and more preferably about 1 to 30 mPa·s. Probably or presumably because of more rapid dissolution in a small quantity of water (e.g., saliva) and formation of a lower viscous aqueous liquid coat, a water-soluble cellulose ether having a lower viscosity can effectively prevent the adhesion of the solid preparation to the inner wall of the oral cavity.

The content of the water-soluble cellulose ether of the whole anti-adhesive layer may be selected from the range of about 20 to 99% by mass (e.g., about 30 to 98% by mass) and may usually be about 50 to 95% by mass (e.g., about 60 to 95% by mass) and preferably about 70 to 90% by mass (e.g., about 75 to 90% by mass).

As far as the anionic polymer can be dissolved in water (e.g., saliva) in an environment of the oral cavity, there are no particular limitations thereon. For example, the anionic polymer may include the water-soluble polymer (an anionic polymer such as a carboxy group-containing polymer, a sulfonic acid group-containing polymer, or a phosphoric acid group-containing polymer) as described as the gel-forming agent for the gel-forming layer. The anionic polymer may form, for example, a salt with an inorganic base [e.g., an alkali metal (such as sodium or potassium) and ammonia] or an organic base [e.g., monoethanolamine, diethanolamine, triethanolamine, and dimethylaminoethanol]. The preferred anionic polymer includes the above-mentioned carboxy group-containing polymer, particularly a (meth) acrylic acid-series polymer comprising a (meth)acrylic acid unit as an essential polymerizable component [a homo- or copolymer of (meth)acrylic acid].

The monomer copolymerizable with (meth)acrylic acid may include the copolymerizable monomer described in the gel-forming agent and may be used alone or in combination. For the (meth) acrylic acid-series polymer, the ratio (mass ratio) of the (meth) acrylic acid (or a salt thereof) relative to the copolymerizable monomer is not particularly limited to a specific one as far as the (meth) acrylic acid-series polymer is water-soluble, and for example, the ratio is the same as that described in the gel-forming agent.

As the (meth)acrylic acid-series polymer, there may be mentioned an acrylic acid-series polymer [for example, a polyacrylic acid,an acrylic acid-alkylacrylatecopolymer (e.g., an acrylic acid-methyl acrylate copolymer and an acrylic acid-ethyl acrylate copolymer), and an acrylic acid-alkyl methacrylate copolymer (e.g., acrylic acid-methyl methacrylate and acrylic acid-ethyl methacrylate)], and amethacrylic acid-series polymer (e.g., methacrylic acid-alkyl acrylate copolymer such as a methacrylic acid-methyl acrylate copolymer or a methacrylic acid-ethyl acrylate copolymer). These (meth)acrylic acid-series polymers may be used alone or in combination. The viscosity of the anionic polymer for a 0.2% by mass aqueous solution is usually the same as the viscosity of the aqueous solution of the above-mentioned gel-forming agent.

Representative examples of the (meth)acrylic acid-series polymer may include a carboxyvinyl polymer (tradename:CARBOPOL),a poly(sodium acrylate),a partially neutralized product of a polyacrylic acid, a methacrylic acid-n-butyl acrylate copolymer, and a methacrylic acid copolymer LD (trade name: EUDRAGIT L-30D55). Among these (meth)acrylic acid-series polymers, the preferred one includes an acrylic acid-series polymer obtained by using acrylic acid as a main monomer, particularly a carboxyvinyl polymer (e.g., CARBOPOL and HIVISWAKO exemplified in the above-mentioned gel-forming agent).

The anionic polymer content of the anti-adhesive layer may be selected from the range in which the anti-adhesive layer can rapidly absorb water to form a liquid coat while preventing the adhesion of the solid preparation to the inner wall of the oral cavity, and may for example be about 0.1 to 50% by mass (e.g., about 1 to 30% by mass) in terms of a solid content or a non-volatile matter. The anionic polymer content of the whole anti-adhesive layer may be about 1 to 25% by mass (e.g., about 2 to 20% by mass) and preferably about 3 to 17% by mass (e.g., about 5 to 15% by mass) in terms of a non-volatile matter.

Depending on the species of the water-soluble cellulose ether and anionic polymer, when the water-soluble cellulose ether and the anionic polymer are the same species as the base material of the gel-forming layer and the gel-forming agent, respectively, the ratio of the water-soluble cellulose ether relative to the anionic polymer in the anti-adhesive layer is usually larger than the ratio of the base material relative to the gel-forming agent (an anionic polymer such as a carboxyvinyl polymer) of the gel-forming layer. The mass ratio of the water-soluble cellulose ether relative to the anionic polymer in terms of a solid content may be selected from the range of about 99.9/0.1 to 75/25 (e.g., about 99/1 to 80/20), and may usually be about 99.9/0.1 to 85/15 (e.g., about 99/1 to 85/15) and preferably about 95/5 to 85/15 (e.g., about 92/18 to 87/13), as the water-soluble cellulose ether/the anionic polymer. The ratio of the water-soluble cellulose ether relative to 100 parts by mass of the anionic polymer may for example be about 100 to 2000 parts by mass (e.g., about 200 to 1500 parts by mass), preferably about 300 to 1200 parts by mass (e.g., about 500 to 1000 parts by mass), and more preferably about 600 to 900 parts by mass.

The anti-adhesive layer is usually employed in a liquid form (such as a solution or a dispersion). The anti-adhesive layer has a higher or lower viscosity depending on the species of the water-soluble cellulose and that of the anionic polymer, so that the anti-adhesive layer cannot be formed smoothly in some cases. Thus, the anti-adhesive layer may contain a viscosity modifier for adjusting the viscosity, for example, a viscosity reducing agent or an auxiliary. As the viscosity modifier (such as the viscosity reducing agent or the auxiliary), there may be mentioned a water-soluble metal compound or an electrolyte, a water-soluble organic solvent, and others. The water-soluble metal compound or the electrolyte may include, for example, an alkali metal compound [for example, a halide (a chloride such as sodium chloride or potassium chloride) ; an inorganic acid salt (a sulfate such as sodium sulfate or potassium sulfate; a carbonate such as sodium carbonate, sodium hydrogencarbonate, or potassium carbonate; a phosphate (for example, a sodium phosphate such as sodium monohydrogenphosphate, sodium dihydrogenphosphate, or trisodium phosphate, and a potassium phosphate such as dipotassium phosphate or potassium dihydrogenphosphate), and an organic acid salt (e.g., sodium acetate, sodium fumarate, sodium lactate, sodium citrate, sodium tartrate, sodium potassium tartrate, and potassium hydrogentartrate)], an alkaline earth metal compound [for example, a halide (a chloride such as magnesium chloride or calcium chloride); an inorganic acid salt (a sulfate such as calcium sulfate or magnesium sulfate; a carbonate such as calcium carbonate; a phosphate such as calciumhydrogenphosphate, calciummonohydrogenphosphate, calcium dihydrogenphosphate, or manganese ammonium phosphate; and a silicate such as magnesium silicate), and an organic acid salt (such as calcium acetate or calcium lactate)], a polyvalent metal salt [for example, a halide (a chloride such as aluminum chloride or zinc chloride); an inorganic acid salt (a sulfate such as aluminum sulfate, aluminum potassium sulfate, or zinc sulfate; a phosphate such as aluminum phosphate; a silicate such as aluminum silicate), an organic acid salt (such as aluminum acetate, zinc acetate, or aluminum lactate)], and others. As the water-soluble organic solvent, for example, there may be mentioned an alcohol such as ethanol or ethylene glycol, a ketone such as acetone, a cyclic ether such as dioxane, a cellosolve such as a methyl cellosolve, and N-methyl-2-pyrrolidone. These viscosity modifiers may be used alone or in combination.

Among these components, a metal salt highly reducing the viscosity of the solution (for example, an alkali metal salt and an alkaline earth metal salt) is used practically. The amount of the viscosity modifier (e.g., the viscosity reducing agent) relative to 100 parts by mass of the total amount of the water-soluble cellulose ether and the anionic polymer may for example be selected from the range of about 0 to 200 parts by mass and may usually be about 1 to 100 parts by mass, preferably about 5 to 50 parts by mass, and more preferably about 10 to 30 parts by mass.

Incidentally, the polyvalent metal salt (an alkaline earth metal salt, a tri- to polyvalent metal salt) may function as a crosslinking agent for the anionicpolymer. When such a polyvalent metal salt is used as the viscosity modifier (e.g., the viscosity reducing agent) for the anti-adhesive layer, the amount of the polyvalent metal salt is smaller than the amount of the crosslinking agent relative to 100 parts by mass of the total amount of the base material and the gel-forming agent in the gel-forming layer. The amount of the polyvalent metal salt in the anti-adhesive layer may for example be about 0 to 2 parts by mass (e.g., about 0.01 to 1.5 parts by mass), preferably about 0.05 to 1 parts by mass, and more preferably about 0.1 to 0.5 parts by mass (e.g., about 0.2 to 0.4 parts by mass) relative to 100 parts by mass of the total amount of the water-soluble cellulose ether and the anionic polymer (e.g., a carboxyvinyl polymer). Incidentally, the ratio of the polyvalent metal salt relative to 100 parts by mass of the anionic polymer (e.g., a carboxyvinyl polymer) may for example be about 0.1 to 10 parts by mass (e.g., about 0.5 to 7.5 parts by mass), preferably about 1 to 5 parts by mass, and more preferably about 1.5 to 3.5 parts by mass (e.g., about 2 to 3 parts by mass).

The anti-adhesive layer may contain the various additives as described above, such as the water absorption promoter (for example, glycerin), the masking agent for masking the taste or smell of the active ingredient, the plasticizer (for example, glycerin triacetate, diethyl phthalate, and triethyl citrate), the antiseptic agent or the preservative (for example, methyl hydroxybenzoate, propylhydroxybenzoate, sodium edetate, potassium sorbate, and sodium dehydroacetate), the antioxidant (such as ascorbic acid or tocopherol acetate), and the coloring agent (for example, titanium oxide, and edible lake coloring agent). The masking agent may include an acidifier or an acidulant (e.g., citric acid, tartaric acid, and fumaric acid), a sweetening agent (e.g., saccharin, glycyrrhizinic acid, aspartame, stevioside, acesulfame potassium, and a saccharide), an algefacient (e.g., menthol, mentha oil, peppermint,andspearmint),anaturalor synthetic flavoring agent (or perfume), and others. Among these masking agents, a saccharide (a sugar such as lactose, white sugar or refined sugar, glucose, or sucrose, a sugar alcohol such as mannitol, sorbitol, or xylitol) is preferred.

These components may also be used alone or in combination. The amount of these components may be not more than 20 parts by mass (e.g., about 0.01 to 15 parts by mass, preferably about 0. 05 to 10 parts by mass, and more preferably about 0.1 to 10 parts by mass) relative to 100 parts by mass of the total amount of the water-soluble cellulose ether and the anionic polymer (in terms of a solid content).

It is sufficient that the anti-adhesive layer covers at least part of the surface of the gel-forming layer (for example, not less than 50% of the surface area of the gel-forming layer (e.g., about 50 to 100%, preferably about 87 to 100%, and more preferably about 90 to 100%)). The anti-adhesive layer practically covers the whole of the gel-forming layer or at least upper and under surfaces thereof. The anti-adhesive layer may cover the surface of the gel-forming layer uniformly or nonuniformly (e.g., scatteringly in a polygonal pattern (e.g., quadrilateral pattern), a circular pattern, or a grid pattern).

In order to easily permeate even a small quantity of water (such as saliva) into the anti-adhesive layer, the thickness of the anti-adhesive layer may be not more than 50 µm (e.g., about 1 to 50 µm, preferably about 5 to 45 µm, and more preferably about 10 to 40 µm).

The total thickness of the gel-forming layer and the anti-adhesive layer may for example be about 5 to 1000 µm, preferably about 10 to 500 µm (e.g., about 15 to 250 µm), and more preferably about 20 to 100 µm (e.g., about 25 to 75 µm). Moreover, the thickness ratio of the gel-forming layer relative to the anti-adhesive layer may be selected from the range of about 5/95 to 95/5 (e.g., about 10/90 to 90/10) and may be about 15/85 to 50/50 and more preferably about 20/80 to 40/60 (e.g., about 20/80 to 30/70), as the gel-forming layer/the anti-adhesive layer. By controlling the thickness ratio of the gel-forming layer relative to the anti-adhesive layer, the gel-forming layer rapidly absorbs water through the anti-adhesive layer and swells to form a gel layer having a significantly improved slipperiness in a short period of time, and the anti-adhesive layer can form an aqueous liquid coat as the surface layer. Probably due to such a structure, the solid preparation (solid preparation for oral administration) can easily be swallowed without adhesion to the inner wall of the oral cavity even in absence of water and improve the ease (or easiness) of taking the preparation significantly.

### [Shape of solid preparation]

It is sufficient that the solid preparation comprises the drug-containing unit and the gel-forming layer, and the adhesive layer is not necessarily required. Moreover, the solid preparation does not necessarily comprise the anti-adhesive layer. In order to prevent the contact of the drug of the drug-containing unit with the acidic component, the solid preparation may have the drug-containing unit covered (or coated) with a covering layer, for example, a nonionic polymer (e,g., a cellulose ether such as an MC, an HPC, or an HPMC) or an enteric base material. Further, if necessary, an enteric coating layer, a gastric-soluble coating layer, a water-insoluble coating layer, or other layers may be formed at an appropriate interlayer of the drug-containing unit, the gel-forming layer, and the anti-adhesive layer. The enteric component may include, for example, an enteric base material described in the above-mentioned drug-containing unit. The gastric-soluble component may include, for example, a gastric-soluble base material described in the above-mentioned drug-containing unit. As the water-insolublecomponent,forexample,theremay mentioned an ethyl cellulose, an ethyl acrylate-methyl methacrylate copolymer, and a lipid.

The solid preparation (or solid preparation for oral administration) of the present invention may be in the form corresponding to the drug-containing unit or in the form in which the gel-forming layer and the anti-adhesive layer are extended from the periphery of the drug-containing unit. Moreover, the solid preparation of the present invention may be a film-covered (or laminate) preparation in the form of a flat shape or a discoid shape, for example, a flat or discoid preparation having the drug-containing unit enclosed (or wrapped) with a film- or sheet-like covering layer(s). The plane shape of the film-covered preparation may for example be a polygon (e.g., a quadrilateral), a circle, and an ellipse. According to the solid preparation of the present invention, the gel-forming layer and the anti-adhesive layer improves the slipperiness in the oral cavity by even a small quantity of water. Therefore, even when the film-covered preparation has a large flat-surface area, the preparation can easily be swallowed. The area of the flat surface of the film-covered preparation is not particularly limited to a specific one, and may be about 0.01 to 10 cm (e.g., about 0.05 to 9 cm², preferably about 0.1 to 8 cm², and about more preferably 0.5 to 7 cm²).

Incidentally, the surface of the solid preparation may be embossed, if necessary. Moreover, if necessary, the surface of the solid preparation may be sugar-coated.

### [Process for producing solid preparation]

The solid preparation of the present invention may be prepared by covering the drug-containing unit with the gel-forming layer, if necessary, through the intermediate layer, and the gel-forming layer may be covered with the anti-adhesive layer. The drug-containing unit can be prepared using the active ingredient and the additive according to a conventional manner (such as granulation or tableting), as described above. Moreover, each layer of the solid preparation can be produced by each applying a coating composition corresponding to each layer to the drug-containing unit sequentially. Each of the coating compositions corresponding to each layer can be prepared by dispersing or dissolving constituents of each layer (for example, the anti-adhesive layer) in a liquid medium such as water (e.g., a purified water) or a lower alcohol (e.g., ethanol), optionally an organic solvent. Incidentally, if necessary, the resulting coating composition (liquid coating composition or coating agent) may be defoamed.

Depending on the dosage form, a method for coating the drug-containing unit with the coating composition may include, for example, a pan coating, a fluidizedbed coating, a tumbling coating, and a tumbling fluidized bed coating. For example, coating (applying), spraying, and impregnation or immersion may be used for coating the drug-containing unit with the coating composition. Incidentally, each coating composition may be coated successively after drying or without drying.

For the preparation of the solid preparation of the present invention, there may be used lamination or stacking of each layer to the drug-containing unit by flow-casting, coating (applying), or other means. For example, the solid preparation of the present invention may be prepared by a process which comprises an optional step for applying an anti-adhesive composition (coating agent) to a releasable (separable) substrate to form an anti-adhesive layer (an anti-adhesive layer forming step), a step for laminating a gel-forming layer on the anti-adhesive layer (a gel-forming layer laminating step), and an optional step for laminating an adhesive layer on the gel-forming layer (an adhesive layer laminating step), and a step for interposing a drug-containing unit between two laminates prepared through these steps and adhering (or bonding) these laminates (an adhering step).

The releasable substrate is not particularly limited to a specific one, and, for example, a glass plate, a plastic film, and a releasable (separable) sheet may be used. If necessary, these releasable substrates may be embossed by a conventional manner.

The anti-adhesive layer, the gel-forming layer, and the adhesive layer can be formed by coating the releasable substrate with each liquid coating composition using a conventional film-forming method (for example, a method using coating (applying) such as flow-casting, or spraying). Incidentally, the adhesive layer may be formed by coating the gel-forming layer partly. Moreover, the anti-adhesive layer is not essentially formed on the whole surface of the gel-forming layer. In order to form the aqueous liquid coat and the gel layer uniformly and improve the ease of swallowing the preparation, the whole surface of the gel-forming layer is practically coated with the anti-adhesive layer.

In the adhering step, a pair of laminates can be adhered (bonded) to each other while interposing the drug-containing unit between these laminates with the gel-forming layers (or adhesive layers) facing each other. The drug-containing unit can be arranged at a predetermined position with the use of a method for positioning the preparation at a predetermined site (or area), coating (applying), spraying, dropping, ink-jetting, screen-printing, or others. Incidentally, when an embossed releasable substrate having the gel-forming layer (or adhesive layer) is used, the drug-containing unit may be placed in a recessed area formed in the gel-forming layer (or adhesive layer).

As a method for adhering the laminates, for example, thermal adhesion (or hot-melting) or other means can be utilized when a heat (or thermal) adhesive is used. The temperature of the thermal adhesion may for example be about 70 to 150°C (e.g., about 75 to 140°C, preferably about 80°C to 130°C, and more preferably about 85 to 120°C).

The solid preparation can be produced by adhering the periphery of the drug-containing unit to prepare a laminate (or laminated product) having the above layers, and then punching out the periphery of the drug-containing unit in a predetermined shape (e.g., a circular shape, an elliptical shape, and a polygonal shape) depending on the shape of the drug-containing unit.

Further, as described above, the present invention includes a method for improving an elution property of a drug from a solid preparation which comprises a drug-containing unit containing a cationic or basic drug, a gel-forming layer containing an anionic or acidic polymer and absorbs water for forming a gel, and if necessary, an intermediate layer interposed between the drug-containing unit and the gel-forming layer, and the method comprises incorporating a pharmaceutically acceptable acidic component in the drug-containing unit or the intermediate layer.

Moreover, as described above, even for a patient with achlorhydria or hypochlohydria, the improved elution property of the cationic drug from the preparation is expected by incorporating the pharmaceutically acceptable acidic component into the drug-containing unit or the intermediate layer of the solid preparation.

### EXAMPLES

Hereinafter, the following examples are intended to describe this invention in further detail and should by no means be interpreted as defining the scope of the invention.

### (Example 1)

### (a) Step for producing anti-adhesive layer

A liquid coating composition A containing constituents of an anti-adhesive layer was prepared as follows.

To 380 parts by mass of purified water, 0.27 parts by mass of calcium chloride (Calcium chloride H, manufactured by Tomita Pharmaceutical Co., Ltd.) as a viscosity reducing agent was added and dissolved by stirring. To this solution was slowly added 10.0 parts by mass of a carboxyvinyl polymer (a polyacrylic acid, CARBOPOL 974P, manufactured by Noveon, viscosity of 0.2% by mass aqueous solution (20°C) : 12100 mPa·s) with stirring, and the mixture was stirred for one hour. The mixture containing each component was heated to 80°C. To the mixture was slowly added 81.63 parts by mass of a hydroxypropylmethyl cellulose (TC-5E, manufactured by Shin-Etsu Chemical Co., Ltd., viscosity of 2% by mass aqueous solution (20°C) : 3 mPa·s) as an anti-adhesive agent with stirring. After the addition, the mixture was stirred for 15 minutes, and the temperature of the mixture was decreased to 30°C, and the mixture was stirred for one hour. To the resulting mixture was added 8.1 parts by mass of glycerin (Japanese Pharmacopoeia, concentrated glycerin, manufactured by Asahi Denka Kogyo K.K.) as a water absorption promoter, and the mixture was stirred for 15 minutes to give a liquid coating composition A.

The liquid coating composition A was fully defoamed. A poly(ethylene terephthalate) film (SP-PET3811, manufactured by LINTEC Corporation), as a releasable substrate, had a releasably treated surface. The liquid coating composition A was spread-coated (spread-applied) on an untreated surface of the film using an applicator with an adjusted gap and dried at 80°C for 10 minutes to form an anti-adhesive layer having a thickness of 28 µm after drying, and a laminate intermediate "a" (the anti-adhesive layer/the releasable substrate) was obtained.

### (b) Step for producing gel-forming layer

A liquid coating composition B containing constituents of a gel-forming layer was prepared as follows.

To 700 parts by mass of purified water, 0.6 parts by mass of calcium chloride (Calcium chloride H, manufactured by Tomita Pharmaceutical Co., Ltd.) as a crosslinking agent was added and dissolved by stirring for 5 minutes. To this solution was slowly added 22.7 parts by mass of a polyacrylic acid (CARBOPOL 974P, manufactured by Noveon, viscosity of 0.2% by pass aqueous solution (20°C) : 12100 mPa·s) with stirring, and the mixture was stirred for one hour. To the mixture was slowly added 68.6 parts by mass of a poly(vinyl alcohol) (GOHSENOL EG05T, manufactured by The Nippon Synthetic Chemical Industry Co., Ltd.) with stirring. After the mixture was stirred for 15 minutes, the mixture containing each component was heated to 80°C and stirred for one hour. Thereafter, the mixture containing each component was cooled to 30°C. To the mixture was added 8.1 parts by mass of glycerin (Japanese Pharmacopoeia, concentrated glycerin, manufactured by Asahi Denka Kogyo K.K.) as a water absorption promoter, and the resulting mixture was stirred for about 15 minutes to give a liquid coating composition B.

The liquid coating composition B was fully defoamed. The liquid coating composition B was spread-coated (spread-applied) on the anti-adhesive layer formed in the step (a) using an applicator with an adjusted gap and dried at 80°C for 5 minutes to give a laminate intermediate "b" (a laminate of the gel-forming layer/the anti-adhesive layer/the releasable substrate) having a gel-forming layer of 9 µm tickness after drying.

### (c) Step for producing intermediate layer

A liquid coating composition C-1 containing constituents of an intermediate layer was prepared as follows.

To 220 parts by mass of water, 26 parts by mass of glycerin (Japanese Pharmacopoeia, concentrated glycerin, manufactured by Asahi Denka Kogyo K.K.) as a plasticizer was slowly added with stirring and dissolved. Thereafter, to the solution was slowly added 74 parts by mass of a polyvinylpyrrolidone (PVP K-90, manufactured by ISP Japan Ltd.) as a base material with stirring. The mixture was stirred for 60 minutes to give a liquid coating composition C-1.

The liquid coating composition C-1 was fully defoamed. The liquid coating composition C-1 was spread-coated (spread-applied) on the gel-forming layer formed in the step (b) using an applicator with an adjusted gap and dried at 80°C for 10 minutes to give a laminate intermediate "c" (a laminate of the intermediate layer/the gel-forming layer/the anti-adhesive layer/the releasable substrate) having an intermediate layer of 93 µm thickness after drying.

### (d) Step for forming drug-containing layer

Citric acid monohydrate (4.2 parts by mass) as an pharmaceutically acceptable acidic component, amlodipine besilate (2.9 parts by mass) as a cationic drug, and a crystalline cellulose (92.9partsbymass) as a base material were fully mixed and dispersed in a mortar. The resulting powder was subjected to tablet compression by a tableting machine. On the other hand, the intermediate "c" (a first intermediate "c") was pressed from the intermediate layer side thereof to form a recessed area having a size capable of accommodating a tablet. The tablet (mass: 120 mg, drug content: 3.47 mg) was accommodated in the recessed area and then covered with a second intermediate "c". The periphery of the intermediate layer of the first intermediate "c" and that of the second intermediate "c" were bonded to each other by thermal-adhering at 100°C under 1 kgf/cm² for 3 seconds. In such a process, a laminate having the releasable substrate/the anti-adhesive layer/the gel-forming layer/the intermediate layer/the tablet/the intermediate layer/the gel-forming layer/the anti-adhesive layer/the releasable substrate in this order, which had the tablet included therein, was prepared. After the both releasable substrates were removed, a circular shape having a diameter of 15 mm was punched out of the laminate to produce a solid preparation (oral administration preparation) having a lamination structure. In the punching of the laminate, the thermally adhered region of the intermediate layers was punched to avoid exposure of the tablet.

### (Example 2)

Citric acid monohydrate (41.7 parts by mass) as a pharmaceutically acceptable acidic component, amlodipine besilate (2.9partsbymass) asabasicdrug, andacrystalline cellulose (55.4 parts by mass) as a base material were fully mixed and dispersed in a mortar. The resulting powder was subjected to tablet compression by a tableting machine for obtaining a tablet. In the same manner as in Example 1 except for using the resulting tablet as a drug-containing layer, a solid preparation (oral administration preparation) was produced.

### (Example 3)

Tartaric acid (4.2 parts by mass) as a pharmaceutically acceptable acidic component, amlodipine besilate (2.9partsbymass) asabasicdrug, andacrystalline cellulose (92.9 parts by mass) as a base material were fully mixed and dispersed in a mortar. The resulting powder was subjected to tablet compression by a tableting machine for obtaining a tablet. In the same manner as in Example 1 except for using the resulting tablet as a drug-containing layer, a solid preparation (oral administration preparation) was produced.

### (Example 4)

Tartaric acid (41.7 parts by mass) as a pharmaceutically acceptable acidic component, amlodipine besilate (2.9partsbymass) asabasicdrug, andacrystalline cellulose (55.4 parts by mass) as a base material were fully mixed and dispersed in a mortar. The resulting powder was subjected to tablet compression by a tableting machine for obtaining a tablet. In the same manner as in Example 1 except for using the resulting tablet as a drug-containing layer, a solid preparation (oral administration preparation) was produced.

### (Example 5)

Amlodipine besilate (2.9 parts by mass) as a basic drug, and a crystalline cellulose (97.1 parts by mass) as a base material were fully mixed and dispersed in a mortar. The resulting powder was subjected to tablet compression by a tableting machine for obtaining a tablet. In the same manner as in Example 1 except for using the following liquid coating composition C-2 as an intermediate layer and the resulting powder as a drug-containing layer, a solid preparation (oral administration preparation) was produced.

### [Liquid coating composition C-2]

To 190 parts by mass of water, 14.2 parts by mass of citric acidmonohydrate as a pharmaceutically acceptable acidic component and 22.3 parts by mass of glycerin (Japanese Pharmacopoeia, concentrated glycerin, manufactured by Asahi Denka Kogyo K.K.) as a plasticizer were slowly added with stirring and dissolved. To this solution was slowly added 63.5 parts by mass of a polyvinylpyrrolidone (PVP K-90, manufactured by ISP Japan Ltd.) as a base material with stirring, and the mixture was stirred for 60 minutes to give a liquid coating composition C-2.

### (Example 6)

In the same manner as in Example 5 except for using the following liquid coating composition C-3 as an intermediate layer, a solid preparation (oral administration preparation) was produced.

### [Liquid coating composition C-3]

To 190 parts by mass of water, 14.2 parts by mass of tartaric acid as a pharmaceutically acceptable acidic component and 22.3 parts by mass of glycerin (Japanese Pharmacopoeia, concentrated glycerin, manufactured by Asahi Denka Kogyo K.K.) as a plasticizer were slowly added and dissolved under stirring. To this solution was slowly added 63.5 parts by mass of a polyvinylpyrrolidone (PVP K-90, manufactured by ISP Japan Ltd.) as a base material with stirring, and the mixture was stirred for 60 minutes to give a liquid coating composition C-3.

### (Comparative Example 1)

Amlodipine besilate (2.9 parts by mass) as a basic drug and a crystalline cellulose (97.1 parts by mass) as a base material were fully mixed and dispersed in a mortar. The resulting powder was subjected to tablet compression by a tableting machine, and a tablet was obtained. In the same manner as in Example 1 except for using the resulting tablet as a drug-containing layer, a solid preparation (oral administration preparation) was produced.

### [Test method]

### [Dissolution rate of drug]

For each solid preparation (oral administration preparation) obtained in Examples and Comparative Examples, the dissolution rate of the drug was measured by a method in accordance with Dissolution Test, the second method (Paddle Method) defined in Japanese Pharmacopoeia 15th edition. Incidentally, water was used as a dissolution medium. After stirring for 60 minutes at the number of revolutions of 150 rpm, a sample was collected and quantitatively analyzed by a high-speed liquid chromatography to determine the dissolution rate of the drug on the basis of the amount of the drug supplied in a production of the oral administration preparation.

### [Adherability evaluation]

The oral cavity of a subj ect was washed by gargling. After 2 minutes, the solid preparation was so put into the oral cavity without water as to be adhered to the palate (or the upper wall of the oral cavity) easily. Whether or not the solid preparation was adhered to the palate was examined. When the solid preparation was adhered to the palate, whether or not the solid preparation was separable from the palate was examined. The adherability was evaluated in accordance with the following 5-level criteria. Incidentally, the test was carried out 5 times, and the average was calculated as a comprehensive evaluation.
1 ··· The whole of one side of the solid preparation (oral administration preparation) was adhered to the palate, and the solid preparation could not be separated from the palate easily.
2 ··· Part of one side of the solid preparation (oral administration preparation) was adhered to the palate, and the adhered region of the solid preparation could not be separated from the palate easily.
3 ··· The whole or part of one side of the solid preparation (oraladministration preparation)wasadheredtothepalate, while the adhered region of the solid preparation could be separated from the palate easily with the tongue.
4 ··· The whole or part of one side of the solid preparation (oraladministration preparation)wasadheredtothepalate, while the solid preparation was separated from the palate immediately.
5 ··· The solid preparation (oral administration preparation) was hardly adhered to the palate.

### [Evaluation of swallowing (evaluation of ease of taking preparation)]

The oral cavity of a subj ect was washed by gargling. After 2 minutes, the solid preparation was put into the oral cavity without water, and swallowed. The degree of swallowing the preparation was evaluated in accordance with the following 5-level criteria. Incidentally, the test was carried out 5 times, and the average was calculated as a comprehensive evaluation. Moreover, whether or not the preparation got stuck in the throat, airway or esophagus when swallowed (the safety of taking the preparation) was also examined.
1 ··· Doesn't swell and gelate, cannot be taken without water.
2 ··· Swells and gelates slightly, but cannot be taken without water.
3 ··· Swells and gelates, but would like to take with water if possible.
4 ··· Swells and gelates slowly, and can be taken without water.
5 ··· Swells and gelates rapidly, and can be taken without water.

The results are shown in Table 1.

[Table 1]

**Table 1**

| | Dissolution rate (%) | Adherability | Swallowing property |
|---|---|---|---|
| Example 1 | 85.8 | 5.0 | 5.0 |
| Example 2 | 95.8 | 5.0 | 5.0 |
| Example 3 | 90.5 | 5.0 | 5.0 |
| Example 4 | 98.1 | 5.0 | 5.0 |
| Example 5 | 84.8 | 5.0 | 5.0 |
| Example 6 | 86.9 | 5.0 | 5.0 |
| Comparative Example 1 | 70.1 | 5.0 | 5.0 |

### INDUSTRIAL APPLICABILITY

Since the solid preparation (oral administration preparation) of the present invention can improve the elution property of the drug, the bioavailability can be improved. Moreover, the solid preparation can easily be swallowed in the presence of a small quantity of water (such as saliva) due to a gel-forming layer thereof. Further, the anti-adhesive layer caneffectivelyprevent the adhesion of the solid preparation to the inner wall of the oral cavity and can significantly improve the comfortability of taking the solid preparation.

### DESCRIPTION OF REFERENCE NUMERALS

- 1: Solid preparation
- 2: Drug-containing unit
- 3: Intermediate layer
- 4: Gel-forming layer
- 5: Anti-adhesive layer

## Claims

1. A solid preparation comprising
a drug-containing unit,
a gel-forming layer for covering the drug-containing unit and forming a gel with absorbing water, and
an intermediate layer interposed between the drug-containing unit and the gel-forming layer;
wherein the drug-containing unit contains a cationic or basic drug, the gel-forming layer contains an anionic or acidic polymer, and the intermediate layer contains a pharmaceutically acceptable acidic component.

2. A solid preparation comprising
a drug-containing unit and
a gel-forming layer for covering the drug-containing unit and forming a gel with absorbing water;
wherein the drug-containing unit contains a cationic or basic drug and a pharmaceutically acceptable acidic component, and the gel-forming layer contains an anionic or acidic polymer.

3. A solid preparation according to claim 1 or 2, wherein the cationic or basic drug comprises an active component having at least one basic group selected from the group consisting of a primary amino group, a secondary amino group, a tertiary amino group, and a basic nitrogen-containing heterocyclic group, or a salt thereof,
the gel-forming layer comprises a water-soluble (meth)acrylic polymer having a carboxyl group or a salt thereof and a crosslinking agent, and
the acidic component has an acid dissociation constant pKa of not more than 4.0.

4. A solid preparation according to any one of claims 1 to 3, wherein the gel-forming layer comprises a carboxyvinyl polymer and a polyvalent metal compound.

5. A solid preparation according to any one of claims 1 to 4, wherein the acidic component comprises at least one member selected from the group consisting of an inorganic acid, an aliphatic polyvalent carboxylic acid, a hydroxycarboxylic acid, and an amino acid.

6. A solid preparation according to any one of claims 1 to 5, wherein the acidic component comprises at least one member selected from the group consisting of succinic acid, maleic acid, fumaric acid, lactic acid, malic acid, tartaric acid, citric acid, and gluconic acid.

7. A solid preparation according to any one of claims 1 to 6, wherein the ratio of the acidic component is 1 to 5000 parts by mass relative to 100 parts by mass of the cationic or basic drug.

8. A solid preparation according to any one of claims 1 to 7, which further comprises a surface layer (anti-adhesive layer) for covering the gel-forming layer directly or indirectly and dissolving in water to prevent adhesion of the solid preparation to an inner wall of an oral cavity.

9. A solid preparation according to any one of claims 1 to 8, which is a film-covered preparation.

10. A method for improving an elution property of a drug from the following solid preparation (1) or (2):
(1) a solid preparation comprising
a drug-containing unit containing a cationic or basic drug,
a gel-forming layer for covering the drug-containing unit and forming a gel with absorbing water, wherein the gel-forming layer contains an anionic or acidic polymer, and
an intermediate layer interposed between the drug-containing unit and the gel-forming layer, or
(2) a solid preparation comprising
a drug-containing unit containing a cationic or basic drug and
a gel-forming layer for covering the drug-containing unit and forming a gel with absorbing water, wherein the gel-forming layer contains an anionic or acidic polymer;
the method comprising incorporating a pharmaceutically acceptable acidic component into the intermediate layer or the drug-containing unit.
